# FASCICULE DE BREVET EUROPEEN

(11) **EP 2 072 077 B1**
(45) Date de publication et mention de la délivrance du brevet: **06.03.2013**
(21) Numéro de dépôt: 08172142.5
(22) Date de dépôt: 18.12.2008
(51) Int. Cl.: A61M 37/00

(54) **Dispositif d'injection d'un implant**
Vorrichtung zur Injektion eines Implantats
Implant injection device

(30) Priorité: 21.12.2007 FR 0760289
(43) Date de publication de la demande: 24.06.2009
(73) Titulaire: Rexam Pharma La Verpillière, 38290 La Verpillière Cedex (FR)
(72) Inventeur: Painchaud, Gaetan, 69340, FRANCHEVILLE (FR); Donnette, Xavier, 38460, SOLEYMIEU (FR); Dugand, Pascal, 38780, ESTRABLIN (FR)
(74) Mandataire: Thiollier, Clémence-Olivia Laure Marie

(56) Documents cités:
- EP-A- 0 531 036
- EP-A- 0 596 162
- EP-A- 0 858 813
- EP-A- 1 666 085
- WO-A-00/38779
- WO-A-99/42148
- WO-A-2006/058745
- NL-A- 8 901 124
- US-A- 4 105 030
- US-A- 5 484 403
- US-A- 5 695 463
- US-B1- 6 478 768
- US-B1- 6 530 896

## Description

La présente invention concerne le domaine technique de l'injection d'un ou plusieurs implants dans le corps d'un sujet. Plus précisément, mais non exclusivement, l'invention concerne l'injection intra-musculaire ou sous-cutanée d'un (ou plusieurs) composé pharmaceutique à l'état solide ou semi-solide, que l'on désigne "implant" dans la suite. Le sujet peut être un mammifère, notamment un être humain. On le désignera "sujet" ou "patient" dans la suite. Cet implant est injecté périodiquement, par exemple tous les mois, dans le corps du sujet, à environ 10 mm (millimètres) de la surface de la peau du sujet, cet implant se dissolvant ensuite dans le corps du sujet, au cours du mois. Selon une autre application possible, l'implant est une puce électronique utilisée pour l'identification d'un être vivant.

On connaît déjà du document EP 1 323 450 un dispositif d'injection de comprimés de médicament, comprenant une aiguille creuse fixée à un cylindre recevant les comprimés. Les comprimés sont maintenus dans le cylindre, avant d'être introduits dans l'aiguille puis dans le corps du sujet. Le maintien dans le cylindre est assuré par des doigts flexibles, qui retiennent les comprimés jusqu'à ce que l'on appuie sur la tige de piston du dispositif, et qui s'effacent pour faire passer les comprimés dans l'aiguille, en déformant les doigts flexibles.

Un inconvénient d'un tel dispositif réside dans le fait que la déformation des doigts flexibles engendre une mise sous contrainte des comprimés. En effet, les comprimés subissent la compression à la fois du piston sur lequel l'utilisateur appuie et des doigts flexibles qui exercent une certaine résistance avant de s'effacer. Or, pour certains implants, il peut être problématique de les mettre sous contrainte. En effet, certains implants sont particulièrement fragiles ou facilement déformables, du fait qu'ils comprennent des matériaux destinés à se dissoudre dans le corps du sujet. Ainsi, la pression exercée sur ces implants peut les endommager. En outre, dans le cas d'un implant déformable, la pression exercée dessus fait augmenter son diamètre, si bien que l'implant reste bloqué à l'intérieur du dispositif.

La présente invention vise notamment à fournir un dispositif garantissant que les implants injectés dans le corps du sujet sont intacts.

A cet effet, l'invention a pour objet un dispositif d'injection d'un implant, comportant :
- un corps de réception de l'implant agencé en amont d'une aiguille d'injection,
- des moyens de retenue de l'implant dans le corps de réception,
- des moyens de désactivation des moyens de retenue,
- les moyens de retenue étant configurés de façon à ne pas exercer de contrainte sur l'implant.
   Ainsi, on prévoit un dispositif garantissant que l'implant ne sera pas détérioré ni déformé à la suite d'une contrainte. En effet, le dispositif comprend des moyens de désactivation aptes à empêcher les moyens de retenue d'abîmer l'implant lorsqu'il est introduit dans l'aiguille.
   On comprendra que "ne pas exercer de contrainte sur l'implant" consiste à ne pas lui faire subir de force susceptible de l'endommager. Ainsi, le dispositif présenté ci-dessus peut exercer une légère pression sur l'implant, mais sans risquer de l'endommager. Par exemple, pour un implant en PLA ou PLGA (dérivés d'acides lactiques et glycoliques), on peut exercer une légère pression sur l'implant, par exemple allant jusqu'à 1 N (Newton) pour un implant très fragile ou très déformable, ou allant jusqu'à 20 N (Newton) pour un implant plus résistant ou moins déformable, sans le dégrader.
   Parmi les avantages de l'invention, on notera que les moyens de retenue permettent de retenir l'implant dans le corps de réception tant que l'aiguille n'est pas enfoncée dans le corps du sujet et qu'aucune pression n'a été exercée sur la tige de piston. Ainsi, le dispositif garantit à l'utilisateur que l'implant ne risque pas de tomber accidentellement du dispositif avant que l'aiguille soit enfoncée dans le corps du sujet. II est particulièrement important de s'assurer que l'implant est bien présent dans le dispositif avant son injection, du fait que les conséquences de la non injection d'un implant, l'utilisateur croyant par erreur l'avoir injecté, peuvent être considérables dans le domaine médical. En outre, il est avantageux que l'implant soit retenu dans des moyens agencés en amont de l'aiguille, et non dans l'aiguille, du fait que l'utilisateur du dispositif peut s'assurer, au moment où l'aiguille est enfoncée dans le corps du sujet, et de préférence même lorsqu'elle est totalement enfoncée, que l'implant est toujours dans le dispositif. Or, si l'implant avait été retenu à l'intérieur de l'aiguille, et non en amont de l'aiguille, l'utilisateur ne pourrait pas vérifier, une fois que l'aiguille est enfoncée dans le corps du sujet, que l'implant est toujours présent dans le dispositif, du fait qu'il ne peut plus voir l'aiguille. Cet avantage est notable du fait qu'un implant mal retenu dans le dispositif pourrait aisément tomber du dispositif, juste avant l'injection, au moment où l'aiguille est dirigée vers le bas.
   L'invention peut en outre comporter l'une ou plusieurs des caractéristiques suivantes.
- Les moyens de désactivation des moyens de retenue sont déclenchés de façon automatique, une fois que l'aiguille est, au moins partiellement, en position d'enfoncement, c'est-à-dire enfoncée dans le corps du sujet. Ainsi, l'utilisateur du dispositif n'a pas besoin d'effectuer une manoeuvre spécifique pour activer la libération de l'implant retenu dans le dispositif, ce qui facilite la manipulation du dispositif au cours de l'injection. En particulier, l'utilisateur n'a pas besoin de changer de main pour mettre en oeuvre le transfert de l'implant dans l'aiguille, le dépôt de l'implant dans le corps du sujet et le retrait de l'aiguille, il ne fait donc pas de geste brusque qui serait douloureux pour un patient.
- Les moyens de désactivation agissent directement sur les moyens de retenue. En d'autres termes, les moyens de désactivation n'agissent pas sur les moyens de retenue par l'intermédiaire de l'implant, si bien que l'on évite les risques d'endommager l'implant.
- Le dispositif comprend un piston et les moyens de désactivation sont déclenchés par ce piston, plus précisément par le passage du piston à une position prédéterminée. Le déclenchement automatique de la désactivation des moyens de retenue se fait donc par exemple lorsque l'utilisateur appuie sur le piston afin d'injecter l'implant, sans qu'il n'ait à faire d'autre geste que d'appuyer sur le piston.
- Les moyens de retenue comportent une butée de retenue de l'implant, pouvant prendre une position activée de retenue avant que l'aiguille ne soit dans la position d'enfoncement, c'est-à-dire avant que l'aiguille ne commence à être enfoncée dans le corps du sujet, et une position désactivée une fois que l'aiguille est dans la position d'enfoncement, c'est-à-dire une fois que l'aiguille est, au moins partiellement, enfoncée dans le corps du sujet. De préférence, cette butée de retenue agit en réaction du poids de l'implant, afin d'empêcher l'implant de passer dans l'aiguille, sous l'effet de la gravité, lorsque le dispositif est dans la position verticale. Cette butée de retenue peut prendre la forme d'une butée s'étendant dans une direction transversale du dispositif, en dessous de l'implant, en particulier entre l'implant et l'extrémité proximale de l'aiguille.
- La butée de retenue est portée par une patte élastique.
- Les moyens de retenue sont portés par un élément de retenue, et la désactivation comprend d'une part une coopération entre l'élément de retenue et le corps de réception de l'implant, et d'autre part une coopération entre l'élément de retenue et le piston.
- Les moyens de désactivation sont aptes à mettre en oeuvre un écartement radial des moyens de retenue. Ainsi, la butée de retenue peut passer dans sa position désactivée à la suite d'un écartement radial, généré par exemple par une rampe ménagée dans le corps de réception de l'implant, la rampe coopérant avec les moyens de retenue lorsque le piston du dispositif est déplacé.
- Le dispositif comporte des moyens de vérification, configurés de façon que l'implant soit visible par un utilisateur lorsqu'il est retenu par les moyens de retenue, notamment lorsque l'aiguille est dans une position non visible par l'utilisateur, par exemple complètement enfoncée dans le corps du sujet.

La présente invention propose en outre un dispositif de rétro-injection d'un implant. La rétro-injection consiste à injecter l'implant au cours du retrait de l'aiguille. Un exemple de dispositif de rétro-injection est décrit dans WO 2006/058745 ou dans WO 00/38779. Les inventeurs proposent de fournir un dispositif agencé de telle sorte que l'utilisateur du dispositif puisse contrôler manuellement le retrait de l'aiguille hors du corps d'un patient. En effet, on cherche à éviter un retrait automatique de l'aiguille, sous l'action unique d'un ressort, du fait que ce retrait automatique peut être douloureux pour le patient pour notamment deux raisons : l'utilisateur qui injecte l'implant peut ne pas être habitué à utiliser un dispositif de rétro-injection, et il arrive en outre que l'aiguille du dispositif de rétro-injection soit particulièrement longue.

A cet effet, l'invention a pour objet un dispositif de rétro-injection d'implant, selon la revendication 1.

Ainsi, on propose un dispositif dans lequel les moyens de protection, généralement un fourreau protecteur, peuvent prendre leur position de sécurité, de façon à empêcher l'aiguille de rester découverte après avoir été introduite dans le corps du patient. Cette position de sécurité est prise de préférence de façon automatique, par exemple par libération des moyens de rappel lorsque le piston du dispositif arrive en fin de course, ces moyens de rappel provoquant un déplacement relatif de l'aiguille et du fourreau, de façon que le fourreau puisse protéger l'aiguille. On propose un mouvement relatif du fourreau et de l'aiguille déclenché de façon automatique sans pour autant que le retrait de l'aiguille hors du corps du patient soit mis en oeuvre par les moyens de rappel seuls, sans possibilité de contrôle du mouvement du dispositif par l'utilisateur. Ainsi, l'utilisateur peut grâce à ce dispositif contrôler le mouvement des moyens de rappel en s'opposant au moins en partie à leur libération, de préférence à partir des moyens de préhension, si bien que l'utilisateur peut stopper le mouvement des moyens de rappel s'il le souhaite, ou encore le ralentir ou l'accélérer. Les moyens de préhension comprennent généralement une butée d'appui des doigts de l'utilisateur, de préférence d'appui de l'index et le majeur de l'utilisateur, agencée à l'extrémité proximale du dispositif. Une fois que l'aiguille est enfoncée dans le corps du patient, le retrait de l'aiguille hors du corps peut être mis en oeuvre, au moins en partie, par un mouvement de recul effectué par les doigts de l'utilisateur, de sorte que la distance entre la peau du patient et les moyens de préhension augmente au cours du retrait de l'aiguille.

Un tel dispositif de rétro-injection propose à l'utilisateur un fonctionnement similaire à celui d'un dispositif d'injection de liquide, muni d'une seringue et d'un fourreau protecteur déclenché automatiquement, tout en offrant les spécificités techniques dues à un dispositif de rétro-injection d'implant sous forme solide ou semi-solide, notamment les moyens nécessaires pour injecter l'implant au cours du retrait de l'aiguille. On notera que la rétro-injection d'implant a pour avantage de ne pas exercer de contrainte sur l'implant au moment où ce dernier sort de l'aiguille pour rester dans le corps du patient, à la différence d'une injection simple où le produit (par exemple un médicament liquide) est poussé à travers la peau du patient. Dans un cas d'injection simple, si l'implant est fragile, il est cassé lorsqu'il est poussé à travers la peau. A la différence, au cours de la rétro-injection, l'implant ne traverse pas la peau, il remplit un vide laissé par l'aiguille au moment de son retrait : l'implant est amené jusqu'à l'extrémité proximale de l'aiguille en subissant le moins de contrainte possible, puis, le mouvement de retrait de l'aiguille hors du corps de patient s'effectue, en laissant l'implant dans le corps du patient, à la même profondeur que lorsqu'il était à l'extrémité distale de l'aiguille. Lorsque ce retrait de l'aiguille est effectué de façon automatique, sous l'action d'un ressort, l'utilisateur peut ne pas contrôler manuellement le retrait de l'aiguille (du fait du mécanisme intérieur de rétro-injection, non accessible depuis l'extérieur du dispositif), ce qui peut le perturber s'il est habitué à utiliser des dispositifs d'injection de médicament liquide. Or, si l'utilisateur n'est pas habitué à utiliser un dispositif de rétro-injection, son geste d'utilisation du dispositif peut être maladroit, et donc douloureux pour le patient. Grâce à l'invention, l'utilisateur peut contrôler manuellement le retrait de l'aiguille hors du corps du patient, comme pour certains dispositifs d'injection de médicament sous forme liquide. Le dispositif de rétro-injection a donc un fonctionnement que l'utilisateur connaît. On notera que le dispositif de rétro-injection selon l'invention assure de préférence une rétro-injection automatique, c'est-à-dire que le transfert de l'implant(s) de l'aiguille vers le patient se fait automatiquement en un seul geste lors de la rétractation de l'aiguille, mais que la rétro-injection peut également être, au moins en partie, manuelle. En effet, au moment où l'implant est positionné à l'extrémité distale de l'aiguille, on peut prévoir que le retrait de l'aiguille, du moins le début du retrait de l'aiguille, se fasse de façon manuelle, par un mouvement de recul des doigts de l'utilisateur. Ce début de retrait de l'aiguille crée un espace dans le corps du patient, dans lequel on peut introduire l'implant, sans exercer de contrainte sur lui, en appuyant sur le piston. Puis, la pression supplémentaire sur le piston peut ensuite libérer automatiquement les moyens de protection de l'aiguille et la suite du retrait de l'aiguille hors du corps du patient. Ainsi, les opérations de transfert de l'implant dans le corps du patient et de protection de l'aiguille peuvent être réalisées simultanément (rétro-injection automatique) ou de façon successive (rétro-injection manuelle).

Par ailleurs, le dispositif comporte des moyens de retenue de l'implant en amont de l'aiguille d'injection. Comme cela a été expliqué plus haut, il est particulièrement avantageux que l'implant soit retenu en amont de l'aiguille et non dans l'aiguille, du fait que l'utilisateur du dispositif peut s'assurer, au moment où l'aiguille est enfoncée dans le corps du sujet, et de préférence même lorsqu'elle est totalement enfoncée, que l'implant est toujours dans le dispositif. Par ailleurs, l'implant peut être plus facile à retenir en amont de l'aiguille que dans l'aiguille. En outre, comme la course de l'implant avant de sortir de l'aiguille est plus longue lorsqu'il est retenu en amont de l'aiguille, l'implant présente moins de risques de sortir du dispositif au moment où l'on va enfoncer l'aiguille dans le corps du patient.

La retenue en amont de l'aiguille peut être effectuée de la façon décrite ci-dessus, en utilisant des moyens de désactivation, ou bien en exerçant une légère pression sur l'implant, de façon à la retenir par frottements, par exemple au moyen de doigts flexibles.

Le dispositif de rétro-injection peut en outre comporter l'une ou plusieurs des caractéristiques suivantes.
- Le dispositif comporte un piston comprenant deux parties séparables, une partie distale et une partie proximale, la partie distale étant destinée à positionner l'implant à affleurement de l'extrémité distale de l'aiguille.
- Le dispositif comporte des moyens escamotables de fixation des moyens de protection aux moyens de préhension. Ainsi, lorsque le dispositif est en position d'injection, les moyens de protection sont solidarisés aux moyens de préhension, et lorsque le dispositif est en position de sécurité, les moyens de protection sont désolidarisés des moyens de préhension. Par exemple, les moyens de fixation escamotables comprennent des moyens d'encliquetage agencés respectivement sur l'extrémité proximale des moyens de protection et sur une partie solidarisée aux moyens de préhension.

L'invention sera mieux comprise à la lecture de la description qui va suivre, donnée uniquement à titre d'exemple et faite en se référant aux dessins dans lesquels :
- la figure 1a est une vue en coupe longitudinale partielle d'une partie d'un dispositif selon un premier mode de réalisation de l'invention, illustrant des moyens de retenue en position activée ;
- la figure 1b est une vue similaire à celle de la figure 1, illustrant les moyens de retenue en cours de désactivation ;
- la figure 1c est une vue similaire à la figure 1, illustrant les moyens de retenue en position désactivée ;
- la figure 2a est une vue de dessus de moyens de retenue d'un dispositif selon un second mode de réalisation de l'invention ;
- la figure 2b est une vue en coupe longitudinale partielle d'une partie des moyens de retenue de la figure 2a ;
- la figure 2c est une vue en coupe longitudinale partielle d'une partie des moyens de retenue de la figure 2a, le plan de coupe étant perpendiculaire au plan de coupe de la figure 2b ;
- la figure 3a est une vue de côté d'un dispositif d'injection selon un troisième mode de réalisation de l'invention, le dispositif étant représenté en position d'injection ;
- la figure 3b est une vue en coupe longitudinale selon la flèche 3b de la figure 3a ;
- la figure 3c est une vue en coupe longitudinale selon la flèche 3c de la figure 3a ;
- les figures 3d, 3e et 3f sont des vues respectivement similaires aux vues 3a, 3b, 3c, le dispositif étant dans sa position de sécurité ;
- les figures 4a à 4f sont des vues respectivement similaires aux figures 3a à 3f d'un dispositif d'injection selon un quatrième mode de réalisation de l'invention ;
- la figure 5 et une vue d'un dispositif selon un cinquième mode de réalisation de l'invention.

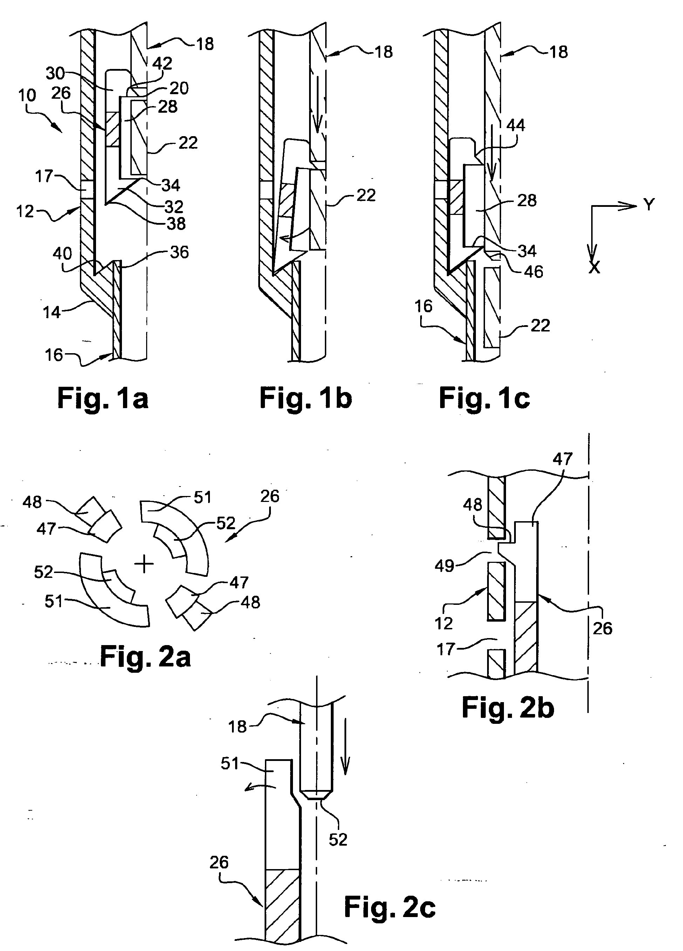

Comme on peut le voir sur les figures 1a à 1c, un dispositif d'injection 10 comprend un corps 12 de réception d'un implant, ce corps 12 comprenant une extrémité distale 14, sur laquelle est fixée, de façon permanente dans cet exemple, une aiguille d'injection 16, destinée à être enfoncée dans le corps d'un sujet. Le dispositif d'injection comporte en outre un piston 18, le piston 18 comprenant une tige, dont l'extrémité proximale est munie d'un poussoir (non représenté), et l'extrémité distale est munie d'une surface de poussée 20. Eventuellement, le piston 18 peut être réalisé en plusieurs segments, comme dans le cas des troisième et quatrième modes de réalisation.

Le dispositif 10 est destiné à recevoir un implant 22 à l'état solide ou semi-solide. Dans cet exemple, l'implant 22 est un unique bloc, mais il pourrait être composé de plusieurs comprimés dissociés, superposés les uns aux autres.

Selon l'exemple décrit, l'aiguille 16 est creuse, elle a un diamètre de 2 mm, et une longueur d'environ 40 mm. Elle est destinée à déposer l'implant 22 à une profondeur d'environ 10 mm de la surface de la peau du sujet. L'implant 22 est particulièrement fragile ou déformable, il est destiné à se dissoudre dans le corps du sujet, par exemple dans une durée de un mois à compter de son injection. Il est réalisé par un procédé d'extrusion ou d'injection, et comprend un matériau support, en PLA ou PLGA, mélangé avec un médicament.

Comme on peut le constater, le corps de réception 12 est agencé juste en amont de l'aiguille d'injection 16. Par ailleurs, le dispositif 10 comporte des moyens de visualisation 17, permettant de vérifier la présence d'un implant 22 dans le dispositif. Ces moyens 17 sont par exemple une lumière ménagée dans le corps de réception 12, agencée de façon que l'on puisse voir l'implant 22 lorsqu'il est reçu dans le dispositif, de façon similaire à celle représentée sur la figure 1a.

Dans la présente demande, on désigne la direction amont en référence à la direction d'injection de l'implant 22. Ainsi, le corps 12 est agencé en amont de l'aiguille 16, c'est-à-dire qu'il est agencé entre l'aiguille 16 et l'extrémité proximale (extrémité opposée à l'aiguille) du dispositif 10.

Le dispositif 10 comporte en outre des moyens 26 de retenue de l'implant 22 dans le corps de réception 12, ces moyens pouvant prendre une position activée, visible sur la figure 1a, et une position désactivée, visible sur la figure 1c. Dans cet exemple, les moyens de retenue sont un élément de retenue 26, de forme générale tubulaire, ajourée. L'élément 26 est monté coulissant à l'intérieur du corps de réception 12. Cet élément 26 comprend un logement tubulaire 28, destiné à recevoir l'implant 22. Ce logement 28 est configuré de façon à ne sensiblement pas exercer de contrainte sur l'implant 22 lorsqu'il est reçu dans l'élément 26, que les moyens de retenue soient en position activée ou en position désactivée. Les moyens de retenue comportent une extrémité proximale 30 et une extrémité distale 32.

L'extrémité distale 32 porte une butée 34 de retenue distale de l'implant, pouvant prendre une position activée de retenue, visible sur la figure 1a, et une position désactivée de retenue, visible sur la figure 1c. Comme on peut le voir sur les figures, la butée de retenue 34 s'étend dans la direction transversale Y du dispositif 10, cette direction Y étant perpendiculaire à la direction longitudinale X du dispositif 10. La butée de retenue 34, lorsqu'elle est en position activée, est configurée pour agir en réaction au poids de l'implant 22 lorsque le dispositif 10 est dans la position verticale, l'aiguille 16 vers le bas, comme cela est dessiné sur les figures, afin d'empêcher l'implant 22 de passer dans l'aiguille 16, sous l'effet de la gravité, lorsque le dispositif est dans la position illustrée sur la figure 1a. Comme on peut le constater, cette butée 34 est agencée en dessous de l'implant 22, plus précisément entre l'implant 22 et l'extrémité proximale 36 de l'aiguille. La butée 34, dans sa position activée, retient donc l'implant 22 dans le logement 28, en amont de l'aiguille 16. L'extrémité distale 32 des moyens de retenue 26 est mobile entre une position activée, visible sur la figure 1a, et une position désactivée visible sur la figure 1c, au moyen par exemple d'au moins une patte élastique portée par cette extrémité 32, cette patte portant la butée 34.

Le dispositif 10 comporte par ailleurs des moyens de désactivation des moyens de retenue distaux 34. Ces moyens de désactivation comprennent une rampe 38, ménagée sur l'extrémité distale de l'élément de retenue 26, solidarisée à la butée 34, cette rampe étant destinée à coopérer avec une rampe 40 ménagée sur l'extrémité distale intérieure du corps de réception 12, de façon que lorsque l'élément de retenue 26 est dans la position activée illustrée sur la figure 1a, le déplacement de cet élément 26 dans la direction X génère une coopération des rampes 38, 40, de manière que la butée de retenue distale 34 prenne sa position désactivée, visible sur la figure 1 c.

L'extrémité proximale 30 de l'élément de retenue comporte par ailleurs une butée 42 de retenue proximale, permettant de retenir l'implant dans le logement 28 lorsque le dispositif 10 est en position verticale, l'aiguille 16 étant vers le haut (position opposée à celle schématisée sur la figure 1a). Comme on peut le voir sur la figure 1a, la butée 42 s'étend dans la direction transversale Y du dispositif 10. Cette butée 42 est portée par une ou plusieurs pattes élastiques portées par l'extrémité proximale 30 de l'élément de retenue 26. L'extrémité 30 est par ailleurs destinée à être traversée par le piston 18, et comporte une surface 44, formant une rampe et destinée à coopérer avec une surface tronconique 46 de l'extrémité distale du piston 18. La rampe 44 est destinée tout d'abord à former une butée pour l'extrémité distale du piston 18, comme on peut le voir sur la figure 1b, puis à s'écarter radialement par déformation élastique, lorsque l'élément 26 est en fin de course dans le corps de réception 12. Les moyens 44, 46 sont des moyens de désactivation de la butée de retenue 42.

On notera que les moyens de retenue 26 sont configurés de façon à ne pas exercer de contrainte sur l'implant 22. En particulier, si ces moyens 26 exercent une pression sur l'implant 22, cette pression n'excède pas une certaine force dépendant de la fragilité ou de la déformabilité de l'implant. Selon l'implant, cette force n'excède pas 1 Newton, ou encore n'excède pas 20 Newton, si bien que la pression n'est pas susceptible d'endommager ou de faire gonfler l'implant 22.

Par ailleurs, l'élément 26 est retenu dans la position illustrée sur la figure 1a grâce à des moyens de maintien de l'élément 26 dans le corps de réception 12. Ce maintien résulte par exemple d'un léger frottement entre l'élément 26 et le corps de réception 12, ce frottement pouvant être effacé lorsque l'utilisateur du dispositif 10 exerce une force de poussée sur le piston 18, comme on peut le voir sur la figure 1b, si bien que l'élément 26 est apte à se déplacer dans la direction X lorsque l'on appuie sur le piston 18.

Le fonctionnement du dispositif d'injection 10 va à présent être décrit.

Avant d'enfoncer l'aiguille 16 dans le corps du sujet, comme on peut le voir sur la figure 1a, les moyens de retenue 26 sont en position activée, les butées de retenue 34, 42 maintenant l'implant à l'intérieur du logement 28. Comme on peut le constater, l'implant 22 est retenu en amont de l'aiguille d'injection 16, et l'on peut vérifier sa présence grâce aux moyens 17 de vérification de la présence de l'implant. Par ailleurs, l'élément 26 est retenu par frottement dans le corps 12 selon la position illustrée sur la figure 1a, grâce aux moyens de maintien décrits plus haut.

Sur la figure 1a, l'aiguille 16 est positionnée de façon à être, au moins partiellement, enfoncée dans le corps du sujet. Dans cet exemple, on considère que l'aiguille 16 de la figure 1a est totalement enfoncée dans le corps du sujet, c'est-à-dire qu'elle est à une profondeur, par rapport à la surface de la peau du sujet, correspondant à la profondeur à laquelle on souhaite placer l'implant 22, par exemple l'extrémité distale de l'aiguille 16 est à 10 mm de la peau du sujet. On notera que, même si l'aiguille 16 est entièrement enfoncée dans la peau, on peut néanmoins vérifier la présence de l'implant 22 grâce aux moyens 17.

On procède ensuite à la libération de l'implant 22, de façon à l'introduire tout d'abord dans l'aiguille 16 (comme on peut le voir sur la figure 1c) puis dans la peau du sujet. Afin de procéder à cette injection, l'utilisateur du dispositif d'implant 10 appuie sur le poussoir du piston 18, ce qui a pour effet de le déplacer dans la direction longitudinale X, vers le bas. Lorsque l'utilisateur pousse sur le piston 18, la surface de butée 46 du piston appuie sur la surface de butée 44, ce qui a pour effet de déplacer l'élément de retenue 26 dans la direction X, comme on peut le voir sur la figure 1 b. En effet, la force de frottement exercée par les moyens de maintien de l'élément 26 dans le corps 12 est inférieure à la poussée exercée par le piston 18 sur la surface de butée 44, si bien que l'élément 26 se déplace dans la direction X lorsque l'on appuie sur le piston 18.

Sous la pression du piston 18, lorsque l'élément 26 arrive en fin de course à l'intérieur du corps de réception 12 (visible sur la figure 1b), les rampes 38 et 40 coopèrent, ce qui a pour effet d'écarter radialement les pattes élastiques de l'extrémité distale 32, et en particulier la butée de retenue 34. Cette butée de retenue 34 étant écartée de l'axe central du dispositif d'injection 10, elle est en position désactivée, si bien qu'elle n'assure plus la retenue de l'implant 22 dans le logement 28. Par ailleurs, comme l'élément de retenue 26 est arrivé en fin de course dans le corps de réception 12, la poussée supplémentaire du piston 18 par l'utilisateur engendre la déformation de l'extrémité proximale 30 de l'élément de retenue 26. Plus précisément, la surface 46 du piston coopère avec la rampe 44 qui se déforme, de façon à laisser le piston 18 traverser l'élément de retenue 26. Le piston 18 peut ainsi pousser l'implant 22 de façon à l'introduire dans l'aiguille 16, comme on peut le voir sur la figure 1c. Dans cette position, l'implant 22 est dans l'aiguille, et peut ensuite être injecté dans le corps du sujet.

Comme on peut le constater, les différents moyens de désactivation, d'une part les rampes 38, 40, d'autre part les rampes 44, 46, sont déclenchés de façon automatique, uniquement une fois que l'aiguille 16 est enfoncée dans le corps du sujet. Ces moyens de désactivation sont déclenchés par le piston 18, plus précisément par le passage du piston d'une position initiale, visible sur la figure 1a, à une position finale, visible sur la figure 1c.

Par ailleurs, les moyens de désactivation 38, 40 ; 44, 46, agissent directement sur les moyens de retenue 26, plus précisément par coopération entre le corps de réception 12 et l'élément de retenue 26 d'une part, et entre le piston 18 et l'élément de retenue 26 d'autre part. En conséquence, ces moyens de désactivation n'agissent pas directement sur l'implant 22, si bien que l'on n'exerce pas de contrainte sur l'implant pour désactiver les moyens de retenue.

On notera que l'invention n'est pas limitée aux modes de réalisation précédemment décrits.

Sur le mode de réalisation des figures 1a à 1c, l'élément 26 est maintenu dans le corps de réception 12 par frottement entre l'élément 26 et le corps 12. Une autre façon avantageuse de maintenir l'élément 26 dans le corps 12 est illustrée sur les figures 2a à 2c. Comme on peut le voir sur la figure 2a, les moyens de retenue 26 de ce mode de réalisation comportent, à leur extrémité proximale 30, quatre pattes élastiques. Deux des pattes élastiques, diamétralement opposées, référencées 47, sont munies d'ergots 48 sur leur surface extérieure, ces ergots étant destinés à coopérer avec une fente 49 réalisée dans le corps de réception 12, comme on peut le voir sur la figure 2b. Ces moyens 48, 49 constituent une butée haute de positionnement de l'élément 26, destinée à assurer son maintien dans une position initiale similaire à celle de la figure 1a, par un encliquetage léger. Cet encliquetage s'efface lorsque le piston 18 s'enfonce, de façon que l'élément 26 se déplace conformément à la figure 1b. Par ailleurs, deux autres pattes 51, diamétralement opposées, sont prévues sur l'extrémité proximale de l'élément 26. Ces pattes 51 sont munies d'ergots intérieurs 52, destinés à former des rampes similaires à la rampe 44, comme on peut le voir sur la figure 2c. Ce mode de réalisation assure un positionnement particulièrement fiable de l'élément 26 dans sa position initiale de la figure 1a.

Par ailleurs, l'élément de retenue est, dans ces exemples, un élément de forme générale tubulaire 26, dont les extrémités distale 32 et proximale 30 comprennent des pattes élastiques. Les moyens de retenue peuvent prendre une multiplicité d'autres formes. Selon un autre exemple, cet élément de retenue 26 pourrait avoir une forme générale tronconique, le diamètre de son extrémité distale 32 étant supérieur au diamètre de son extrémité proximale 30, de façon à garantir de façon plus sûre la coopération respective des éléments 38, 40, 44, 46. Par ailleurs, cet élément 26 n'est pas forcément une pièce distincte des autres pièces du dispositif d'implant, les moyens de retenue pouvant être ménagés sur une ou plusieurs pièces du dispositif d'injection 10 ayant une autre fonction que la retenue de l'implant 22.

Par ailleurs, le dispositif d'injection décrit dans ce qui précède peut être un dispositif de rétro-injection, comme cela va être décrit dans la suite.

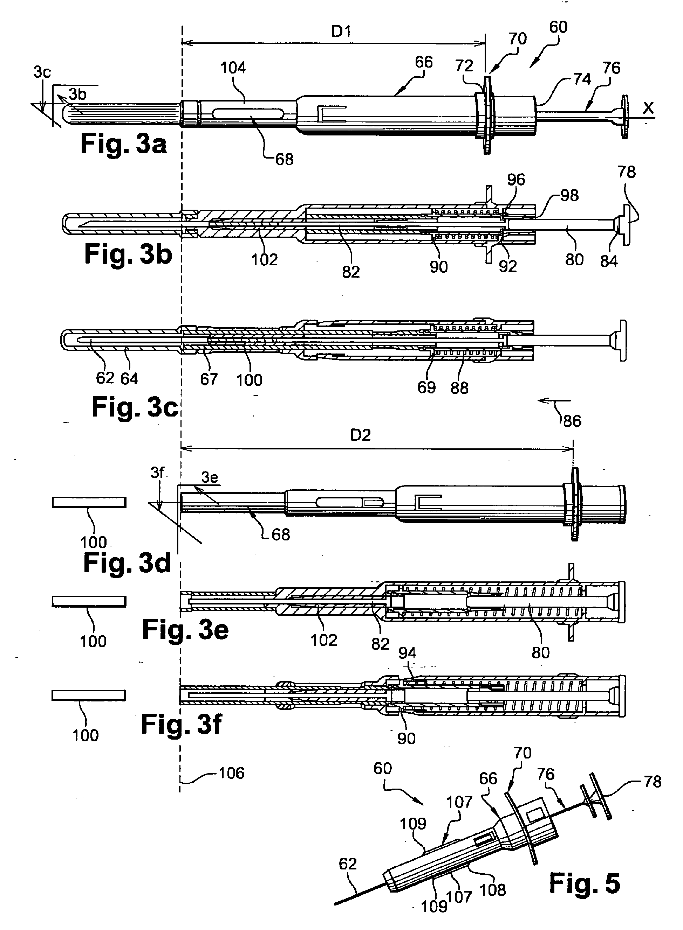

Un dispositif d'injection 60 selon un troisième, un quatrième et un cinquième modes de réalisation va à présent être décrit, en référence aux figures 3a à 5.

Comme on peut le voir sur les figures 3a à 3f, le dispositif 60 selon le troisième mode de réalisation comprend une aiguille d'injection 62, recouverte, avant le début de l'utilisation du dispositif 60, par un capuchon de protection 64. Le dispositif 60 comporte en outre un corps intermédiaire 66 et des moyens de protection 68, que l'on appelle dans la suite "fourreau de protection 68", destinés à recouvrir l'aiguille après utilisation du dispositif, comme on peut le voir sur les figures 3d à 3f. Dans ce mode de réalisation, le corps intermédiaire 66 est extérieur, et le fourreau 68 est agencé à l'intérieur du corps intermédiaire 66, coaxialement par rapport à l'axe longitudinal X du dispositif.

Dans ce mode de réalisation, le fourreau 68 comporte deux parties, à savoir une partie distale 67, assurant la fonction de protection de l'aiguille lorsque le dispositif est en position de sécurité, et une partie proximale 69, rapportée sur la partie distale 67, assurant notamment une fonction de maintien du fourreau en position d'injection, et également de maintien du fourreau en position de sécurité.

Le corps intermédiaire 66 porte des moyens de préhension 70 du dispositif. Les moyens de préhension 70 comprennent une butée 72 d'appui de doigts de l'utilisateur, en particulier de l'index et du majeur de l'utilisateur. Dans cet exemple, les moyens de préhension 70 comprennent une collerette de révolution ménagée en saillie du corps intermédiaire 66, au voisinage de son extrémité proximale 74. Le dispositif 60 comporte en outre un piston 76. Comme on peut le voir notamment sur les figures 3b et 3e, le piston 76 comprend un poussoir 78, destiné à servir d'appui au pouce de l'utilisateur, une partie proximale 80, et une partie distale 82, séparable de la partie proximale 80. Le piston 76 comprend en outre des moyens 84 de libération de moyens de rappel, comme cela est décrit dans la suite, ces moyens 84 comprenant une forme en entonnoir, ménagée entre la partie proximale 80 et le poussoir 78, destinée à former une rampe lorsque le piston est déplacé dans la direction illustrée par la flèche 86.

Le dispositif 60 comporte en outre des moyens de rappel 88, agencés entre le corps intermédiaire 66 et le fourreau 68, en étant comprimés lorsque le dispositif 60 est en position d'injection, et pouvant prendre une position étendue, ou de repos, lorsque le dispositif est en position de sécurité, comme on peut le voir sur les figures 3d à 3f. Les moyens 88 sont un ressort en compression.

Comme on peut le voir sur la figure 3b, les moyens 88 sont maintenus à l'état comprimé, dans la position d'injection, au moyen d'une butée distale 90, ménagée sur le fourreau 68, et d'une butée proximale 92, ménagée sur le corps intermédiaire 66. Comme on peut le voir sur la figure 3f, la butée distale 90 du fourreau est déplaçable en translation jusqu'à une position de sécurité. Dans ce mode de réalisation, la butée 90 assure également une fonction de blocage du fourreau 68 en position de sécurité, en coopérant par encliquetage avec des moyens tels que des pattes élastiques 94 à débattement radial, portées par le corps intermédiaire 66.

Le fourreau 68 est maintenu en position d'injection grâce à des ergots escamotables 96 ménagés sur sa partie proximale 69, plus précisément au voisinage de l'extrémité proximale de cette partie proximale 69. La partie proximale 69 comporte par ailleurs des moyens 98 de libération des moyens de rappel 88, destinés à coopérer avec les moyens 84 du piston 76.

Comme on peut le voir sur les figures 3a à 3c, le fourreau 68 et le corps intermédiaire 66 forment ensemble, à leur extrémité distale, un corps de réception d'un (ou plusieurs) implant 100, agencé en amont de l'aiguille 62, destiné à être injecté dans le corps d'un patient. Le dispositif 60 comporte en outre des moyens 102 de retenue de l'implant 100 dans le corps de réception. Dans l'exemple des figures 3a à 3f, les moyens de retenue 102 assurent leur fonction de retenue au moyen de doigts flexibles exerçant une légère pression sur l'implant 102. Néanmoins, ces moyens de retenue 102 peuvent être remplacés par des moyens de retenue tels que présentés ci-dessus, par exemple par les moyens de retenue 26, désactivables par des moyens de désactivation de façon à ne sensiblement pas exercer de contrainte sur l'implant 100.

Le corps intermédiaire 66 comporte par ailleurs des moyens de vérification 104, composés dans cet exemple d'une fenêtre ménagée dans le corps 66, permettant de visualiser que l'implant 100 est effectivement retenu par les moyens de retenue 102.

Le fonctionnement du dispositif des figures 3a à 3f va à présent être décrit.

Avant l'utilisation du dispositif de rétro-injection 60, l'implant 100 est présent dans le dispositif, sa présence pouvant être vérifiée grâce aux moyens 104. L'injection commence par un retrait du capuchon de protection 64, de façon à laisser découverte l'aiguille 62, afin que l'utilisateur du dispositif la fasse pénétrer dans le corps du patient dans lequel on souhaite injecter l'implant 100. A cet effet, l'utilisateur saisit le dispositif 60 entre son doigt index et son doigt majeur, ces deux doigts prenant appui sur les moyens de préhension 72, le pouce de l'utilisateur étant appelé à pousser sur le poussoir 78 dans le sens de la flèche 86. On notera que l'aiguille est totalement découverte avant d'être introduite dans le corps du patient, si bien que l'utilisateur voir précisément ce qu'il fait, et contrôle manuellement l'enfoncement de l'aiguille, comme pour un dispositif d'injection de liquide. Une fois l'aiguille complètement introduite dans le corps du patient, dont la peau est représentée par la ligne 106 en pointillé, l'utilisateur pousse sur le piston 76, en appuyant sur le poussoir 78. Le déplacement du poussoir 78 entraîne le déplacement de la partie proximale 80 et de la partie distale 82 du piston, qui entraîne elle-même le déplacement de l'implant 100 dans l'aiguille 62. Une fois l'implant 100 positionné à affleurement de l'extrémité distale de l'aiguille 62, le piston 76 arrive à une position dans laquelle les moyens 84 sont aptes à coopérer avec les moyens 98. Plus précisément, la rampe 84 du piston 76 entraîne l'écartement radial de la rampe 98 du fourreau 68, cet écartement entraînant l'escamotage des ergots 96, donc la libération des moyens de rappel 88. II résulte de cet escamotage que les ergots 96 passent au-delà de la butée de retenue 92 du corps intermédiaire 66, dans le sens de la flèche 86, si bien que la butée de retenue 90 du fourreau 68 se déplace, sous l'effet des moyens de rappel 88, dans la direction de la flèche 86. Ce déplacement a pour effet de déplacer le fourreau 68 vers la direction 86, ce qui entraîne, d'une part, le déplacement de la partie 82 du piston, préalablement solidarisée au fourreau 68 (par frottement ou par encliquetage), à l'intérieur de l'aiguille, afin de maintenir les implants en place, et d'autre part la mise en place de la position de sécurité par le fourreau 68, visible sur les figures 3d à 3f, si bien que le fourreau 68 peut recouvrir l'aiguille 62 au fur et à mesure qu'elle est retirée du corps du patient. Une fois que l'aiguille est totalement retirée, le fourreau 68 la recouvre totalement, et les butées 90 coopèrent avec les moyens 94 du corps 66, de façon à bloquer le dispositif en position de sécurité. Du fait que la partie 82 du piston pousse automatiquement sur l'implant, sous l'effet de la libération du fourreau, pour le maintenir dans le corps de l'utilisateur, on parle de rétro-injection automatique.

On notera que la rétro-injection peut être, de manière alternative, manuelle. Dans ce cas, le piston 76 est en une seule partie, non solidarisable au fourreau 68, et l'injection de l'implant a lieu avant l'escamotage des ergots 96. En effet, au moment où l'implant 100 est à l'extrémité distale de l'aiguille 62, l'utilisateur peut effectuer un léger mouvement de recul du dispositif 60, ce qui génère un début de retrait de l'aiguille, et laisse ainsi de la place dans le corps du patient pour introduire l'implant 100 sans contrainte. Après ce léger recul, l'utilisateur peut appuyer à nouveau sur le poussoir 78, ce qui permet au piston 76 (non solidaire du fourreau) de pousser l'implant 100 dans le corps du patient. Puis, l'enfoncement supplémentaire du poussoir 78 entraîne l'escamotage des ergots 96, donc la libération automatique des moyens de protection 68.

Comme on peut le constater sur les figures, la distance entre les moyens de préhension 70 et la peau du patient 106 a été augmentée de la valeur D₁, lorsque le dispositif est en position d'injection à la valeur D₂, lorsque le dispositif est en position de sécurité. Ainsi, le dispositif 60 permet la rétro-injection de l'implant 100, c'est-à-dire que l'implant 100 est injecté, plus précisément reste en position dans le corps du patient, au moment du retrait de l'aiguille 62 hors du corps du patient. Outre la rétro-injection, on propose une protection automatique de l'aiguille par le fourreau 68, au moment de son retrait, l'aiguille n'étant pas laissée découverte, tout en offrant la possibilité à l'utilisateur de contrôler manuellement le retrait de l'aiguille à l'aide des moyens 70. En effet, pour retirer l'aiguille 62 de la peau 106 du patient, les doigts de l'utilisateur bougent, en reculant vers l'arrière. Si l'utilisateur ne contrôle pas ses doigts, ils sont entraînés par le mouvement des moyens de rappel 88 qui déclenchent la sortie du fourreau 68 hors du corps intermédiaire 66. Dans ce cas, le retrait de l'aiguille se fait automatiquement, sans contrôle manuel de l'utilisateur. Néanmoins, l'utilisateur peut contrôler manuellement le retrait de l'aiguille, en contrôlant le mouvement des moyens 88 par l'intermédiaire des moyens 70. En effet, l'utilisateur peut par exemple ralentir ou stopper le mouvement des moyens 88, en exerçant une force sur les moyens de préhension 70 allant à l'encontre de la force exercée par les moyens de rappel 88.

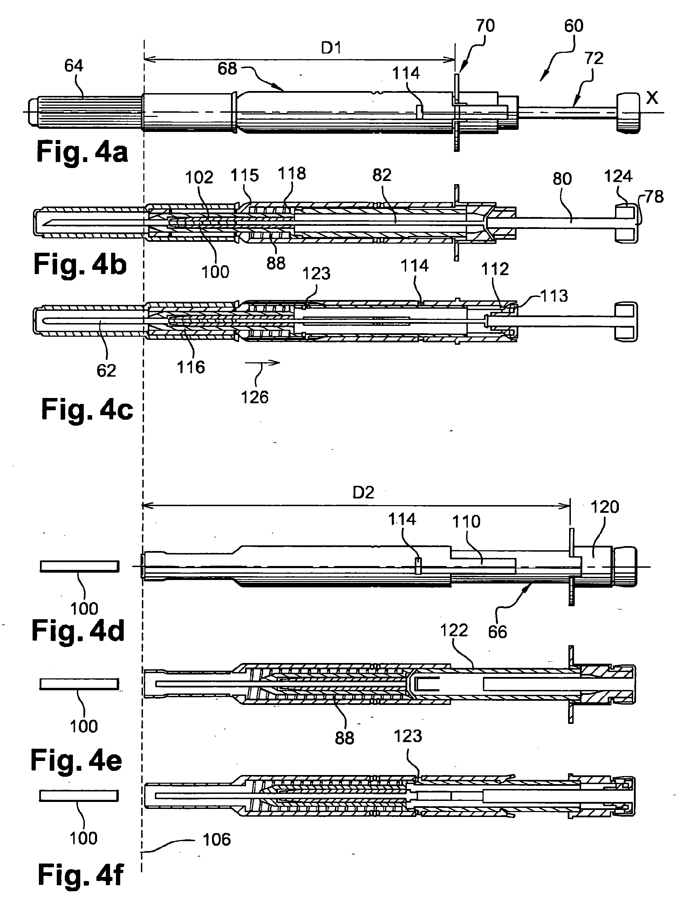

Le dispositif de rétro-injection 60 illustré sur les figures 4a à 4f a une structure différente du dispositif des figures 3a à 3f, mais un fonctionnement similaire.

Dans ce mode de réalisation, le fourreau 68 est disposé à l'extérieur, et le corps intermédiaire 66 est agencé, de façon coaxiale par rapport à l'axe X, à l'intérieur du fourreau 68.

Dans ce mode de réalisation, le fourreau 68 porte, à son extrémité proximale, des moyens escamotables 110, composés dans l'exemple de deux pattes élastiques 110, munies chacune d'ergots escamotables 112, destinées à coopérer avec le corps intermédiaire 66. Les pattes escamotables 110 comportent par ailleurs des rampes 113, destinées à coopérer avec le piston 72. Le fourreau 68 comporte par ailleurs des moyens 114 de blocage du fourreau en position de sécurité, ces moyens 114 étant dans l'exemple destinés à coopérer avec le corps intermédiaire 66.

Par ailleurs, le fourreau 68 comporte une butée 115 destinée à servir d'appui aux moyens de rappel 88. Le corps intermédiaire 66 comporte un corps 116 de réception de l'implant 100, l'implant étant retenu grâce aux moyens de retenue 102. Par ailleurs, le corps intermédiaire 66 comporte une butée 118 destinée à servir d'appui à l'extrémité proximale des moyens de rappel 88. Dans ce mode de réalisation, le corps intermédiaire 66 est composé de deux parties, à savoir une partie proximale 120, portant les moyens de préhension 70, rapportée sur une partie distale 122, comprenant le corps de réception de l'implant 100. Le corps intermédiaire 66 est muni de moyens 123 de blocage du fourreau 68 en position de sécurité, en l'occurrence des ergots 123 destinés à coopérer avec les fentes 114.

Dans ce mode de réalisation, le poussoir 78 du piston 72 est pourvu de moyens 124 de libération des moyens de blocage 112, grâce à une forme de capuchon du poussoir 78.

Le fonctionnement du dispositif 60 des figures 4a à 4f est similaire à celui du dispositif des figures 3a à 3f, les étapes principales étant décrites dans la suite.

L'utilisateur commence par ôter le capuchon de protection 64, puis à introduire l'aiguille 62 dans le corps du patient. Puis, l'utilisateur appuie sur le poussoir 78, ce qui a pour effet de déplacer les tiges 80 et 82 du piston 72, donc l'implant 100 qui est introduit dans l'extrémité distale de l'aiguille 62. Lorsque le piston 72 arrive en fin de course, les moyens 124 de libération coopèrent avec la rampe élastique 113, de façon à écarter radialement les pattes élastiques 110 et à escamoter ainsi les ergots 112. Une fois les ergots 112 escamotés, les moyens de rappel 88 sont libérés et peuvent s'étendre dans la direction indiquée par la flèche 126. Cette libération de moyens de rappel 88 a pour effet de déplacer le corps intermédiaire 66 dans la direction de la flèche 126, et donc de faire protéger l'aiguille 62 par le fourreau 68, comme on peut le voir sur les figures 4d à 4f. Ensuite, le dispositif est maintenu en position de sécurité, par coopération des moyens 114 et 123.

De même que pour le troisième mode de réalisation, on constate que l'utilisateur peut contrôler manuellement le retrait de l'aiguille hors de la peau du patient 106.

Le mode de réalisation de la figure 5 est relativement proche de celui des figures 3a à 3f. Néanmoins, outre les moyens de préhension 70 à l'extrémité proximale du corps intermédiaire 66, le dispositif 60 comporte des moyens 107 d'indication de préhension distale du dispositif, la préhension distale étant destinée à faciliter l'enfoncement de l'aiguille dans le corps du patient. Ces moyens 107 sont agencés sur le corps 66, au voisinage de son extrémité distale. Ils peuvent être composés d'une indication visuelle, d'une protubérance (ce qui est le cas sur la figure 5), ou encore d'une surface grainée. Ils sont destinés à indiquer à l'utilisateur une zone distale que l'utilisateur peut utiliser pour mettre en oeuvre l'injection.

Bien sûr, de tels moyens d'indication 107 peuvent être prévus sur tous les autres modes de réalisation. Dans le cas du mode de réalisation des figures 4, les moyens 107 pourraient être prévus sur le fourreau 68.

Quelques précisions sur le fonctionnement du dispositif de la figure 5, applicables également aux autres modes de réalisation, vont à présent être décrites. Avant de commencer à piquer le patient, le dispositif 60 est dans la configuration de la figure 5. Pour introduire l'aiguille 62 dans la peau, l'utilisateur saisit le dispositif 60 par sa partie distale, en tenant de préférence la zone d'indication 107 entre les doigts de sa main droite s'il est droitier. En effet, il peut être plus pratique à l'utilisateur d'introduire l'aiguille en tenant le dispositif 60 au niveau de cette zone 107, du fait que le dispositif 60 peut être relativement long (on peut avoir par exemple une longueur de 200 mm entre l'extrémité de l'aiguille 62 et le poussoir 78). Plus précisément, l'utilisateur tient avec sa main droite l'extrémité proximale 108 de la zone d'indication 107. De son autre main, la main gauche, l'utilisateur peut pincer le corps du patient, afin de créer une surface dure pour introduire l'aiguille. Une fois que l'aiguille est totalement enfoncée dans le corps du patient, l'utilisateur peut arrêter de pincer le corps du patient, et saisir, avec sa main gauche libérée, l'extrémité distale 109 de la zone 107, de façon à libérer sa main droite qui lâche l'extrémité 108 et peut donc saisir les moyens de préhension 70 afin d'activer le piston 76 et procéder aux étapes d'injection et de retrait de l'aiguille. Dans le mode de réalisation de la figure 5, le piston 76 est plutôt en une seule partie, et la rétro-injection est de préférence manuelle.

On notera que les modes de réalisation du dispositif de rétro-injection 60 peuvent prendre d'autres formes que celles décrites. Notamment, les modes de réalisation peuvent être combinés avec l'un ou l'autre des autres modes de réalisation.

On notera que les dispositifs des troisième quatrième et cinquième modes de réalisation (figures 3a à 5) sont des dispositifs de rétro-injection d'implant, de préférence de rétro-injection automatique, mais pouvant également mettre en oeuvre une rétro-injection manuelle, comme cela a été décrit. Ainsi, on propose un dispositif muni de moyens de protection 68 déclenchés de façon automatique, évitant des contaminations par l'aiguille, et assurant la rétro-injection de façon manuelle ou automatique, garantissant que l'implant est injecté sans subir de contrainte. Que la rétro-injection soit manuelle ou automatique, l'invention propose de contrôler manuellement le déplacement des moyens de protection 68, depuis les moyens de préhension 70. Dans le cas d'une rétro-injection manuelle, l'utilisateur procède lui-même à l'injection de l'implant, en reculant l'aiguille à l'intérieur du corps du patient et en exerçant une pression sur le piston avant la libération automatique des moyens de protection. Dans le cas d'une rétro-injection automatique, l'utilisateur ne se soucie pas de l'injection de l'implant, cette dernière étant mise en oeuvre lors de la libération des moyens de protection, grâce à une solidarisation préalable du fourreau et de la partie distale 82 du piston, de manière que le déclenchement du fourreau entraîne la partie 82, qui accompagne donc le transfert des implants vers la peau, lorsque l'aiguille est retirée du corps.

Un fonctionnement similaire peut être envisagé pour un dispositif d'injection autre qu'un dispositif de rétro-injection.

## Revendications

1. Dispositif de rétro-injection (60) d'un implant (22, 100) comprenant :
- des moyens (70) de préhension du dispositif par un utilisateur,
- une aiguille d'injection (62),
- des moyens (102) de retenue de l'implant en amont de l'aiguille d'injection (62),
- des moyens nécessaires pour injecter l'implant (22, 100) au cours du retrait de l'aiguille,
- des moyens de protection (68) aptes à prendre une position d'injection, laissant découverte l'aiguille (62), et une position de sécurité, recouvrant l'aiguille (62) après l'utilisation du dispositif,
- des moyens de rappel (88) aptes à entraîner les moyens de protection (68) jusqu'à leur position de sécurité, le mouvement des moyens de rappel étant contrôlable par l'utilisateur.

2. Dispositif selon la revendication précédente, comportant un piston (76) comprenant deux parties (80, 82) séparables, une partie distale (82) et une partie proximale (80), la partie distale étant destinée à positionner l'implant (100) à affleurement de l'extrémité distale de l'aiguille (62).

3. Dispositif selon l'une quelconque des revendications précédentes, comportant des moyens escamotables (110) de fixation des moyens de protection (68) à des moyens de préhension (70) du dispositif.

4. Dispositif selon l'une quelconque des revendications précédentes, dans lequel les moyens de retenue assurent leur fonction de retenue au moyen de doigts flexibles exerçant une légère pression sur l'implant.

5. Dispositif selon l'une quelconque des revendications 1 à 3 comportant :
- un corps (12) de réception dé l'implant (22, 100), agencé en amont d'une aiguille d'injection (16),
- des moyens (26, 102) de retenue de l'implant (22) dans le corps de réception (12),
- des moyens (38, 40, 44, 46) de désactivation des moyens de retenue (26),
- les moyens de retenue (26, 102) étant configurés de façon à ne pas exercer de contrainte sur l'implant (22).

6. Dispositif selon la revendication 5, comprenant un piston (18), et dans lequel les moyens de désactivation (38, 40 ; 44, 46) sont déclenchés par ce piston (18), plus précisément par le passage du piston (18) à une position prédéterminée.

7. Dispositif selon l'une quelconque des revendications 5 à 6, dans lequel les moyens de retenue (26) comprennent une butée (34, 42) de retenue de l'implant, pouvant prendre une position activée de retenue, avant que l'aiguille (16) ne soit dans la position d'enfoncement, et une position désactivée, une fois que l'aiguille est dans la position d'enfoncement.

8. Dispositif selon la revendication précédente, dans lequel la butée de retenue (34, 42) est portée par une patte élastique (30, 32).

9. Dispositif selon l'une quelconque des revendications 5 à 8, dans lequel les moyens de retenue sont portés par un élément de retenue (26), et la désactivation comprend, d'une part, une coopération entre l'élément de retenue (26) et le corps de réception (12) de l'implant, et d'autre part une coopération entre l'élément de retenue (26) et le piston (18).

10. Dispositif selon l'une quelconque des revendications 5 à 9, dans lequel les moyens de désactivation (38, 40 ; 44, 46) sont configurés pour mettre en oeuvre un écartement radial des moyens de retenue (26).

11. Dispositif selon l'une quelconque des revendications 1 à 10, comportant des moyens de vérification (17, 104), configurés de façon que l'implant (22) soit visible par un utilisateur lorsqu'il est retenu par les moyens de retenue (26, 102), notamment lorsque l'aiguille (16) est dans une position non visible par l'utilisateur.

## Claims

1. Device for back-injection (60) of an implant (22, 100) comprising:
- means (70) for a user to hold the device,
- an injection needle (62),
- means (102) for retaining the implant before the injection needle (62),
- means required for injecting the implant (22, 100) during withdrawal of the needle,
- protective means (68), capable of adopting an injection position leaving the needle (62) uncovered and a safety position covering the needle (62) after use of the device,
- return means (88) designed to move the protective means (68) into their safety position, the movement of the return means being controllable by the user.

2. Device according to the preceding claim, comprising a plunger (76) comprising two separable parts (80, 82), a distal part (82) and a proximal part (80), the distal part being intended to position the implant (100) flush with the distal end of needle (62).

3. Device according to any of the preceding claims, comprising retractable means (110) for securing the protective means (68) to the holding means (70) of the device.

4. Device according to any of the preceding claims, wherein the retaining means perform their retaining function with flexible fingers exerting light pressure on the implant.

5. Device according to any of claims 1 to 3 comprising:
- a housing (12) for receiving the implant (22, 100) located before an injection needle (16),
- means (26, 102) for retaining the implant (22) in the receiver housing (12),
- means (38, 40, 44, 46) for deactivating the retaining means (26),
- the retaining means (26, 102) being configured so as not to exert any stress on the implant (22).

6. Device according to claim 5, comprising a plunger (18), wherein the deactivating means (38, 40, 44, 46) are operated by this plunger (18), more precisely by the movement of plunger (18) into a pre-determined position.

7. Device according to any of claims 5 to 6, wherein the retaining means (26) comprise a stop (34, 42) for retaining the implant, which can adopt an activated retaining position before the needle (16) is in the insertion position and a deactivated position once the needle is in the insertion position.

8. Device according to the preceding claim, wherein the retaining stop (34, 42) is supported by a rubber tab (30, 32).

9. Device according to any of claims 5 to 8, wherein the retaining means are supported by a retaining element (26) and the deactivation comprises, firstly, an interaction between the retaining element (26) and the implant receiver housing (12) and, secondly, an interaction between the retaining element (26) and the plunger (18).

10. Device according to any of claims 5 to 9, wherein the deactivation means (38, 40; 44, 46) are configured to shift the retaining means (26) radially.

11. Device according to any of claims 1 to 10, comprising verification means (17, 104) configured so that the implant (22) is visible to a user when it is held by the retaining means (26, 102), especially when the needle (16) is in a position not visible to the user.

## Patentansprüche

1. Vorrichtung (60) zum Retro-Injizieren eines Implantats (22, 100) mit:
Einrichtungen (70) zum Ergreifen der Vorrichtung durch einen Benutzer,
einer Injektionsnadel (62),
Einrichtungen (102) zum Speichern des Implantats oberhalb der Injektionsnadel (62), Einrichtungen, die zum Injizieren des Implantats (22, 100) beim Einziehen der Nadel benötigt werden,
Schutzeinrichtungen (68), die dazu dienen, eine Injektionsposition, in der die Nadel (62) offenliegt, und eine Sicherheitsposition einzunehmen, in der die Nadel (62) nach der Benutzung der Vorrichtung bedeckt ist,
Rückholeinrichtungen (88), die dazu dienen, die Schutzeinrichtungen (68) in ihre Sicherheitsposition zu bringen, wobei die Bewegung der Rückholeinrichtungen durch den Benutzer steuerbar ist.

2. Vorrichtung nach dem vorangehenden Anspruch, die einen Kolben (76) mit zwei trennbaren Teilen (80, 82) umfasst, nämlich einem entfernten (82) und einem benachbarten (80) Teil, wobei der entfernte Teil dazu dient, das Implantat (100) genau am entfernten Ende der Nadel (62) zu positionieren.

3. Vorrichtung nach einem der vorangehenden Ansprüche mit einziehbaren Einrichtungen (110) zum Fixieren der Schutzeinrichtungen (68) an den Greifeinrichtungen (70) der Vorrichtung.

4. Vorrichtung nach einem der vorangehenden Ansprüche, bei der die Speicherfunktion der Speichereinrichtungen mittels flexibler Finger sichergestellt wird, die einen leichten Druck auf das Implantat ausüben.

5. Vorrichtung nach einem der Ansprüche 1 bis 3 mit:
einem Gehäuse (12) zur Aufnahme des Implantats (22, 100), das oberhalb einer Injektionsnadel (16) angeordnet ist,
Einrichtungen (26, 102) zum Speichern des Implantats (22) in dem Aufnahmegehäuse (12),
Einrichtungen (38, 40, 44, 46) zum Deaktivieren der Speichereinrichtungen (26),
wobei die Speichereinrichtungen (26, 102) derart ausgelegt sind, dass sie keine Einschränkung in Bezug auf das Implantat (22) erforderlich machen.

6. Vorrichtung nach Anspruch 5 mit einem Kolben (18), bei der die Deaktivierungseinrichtungen (38, 40; 44, 46) durch den Kolben (18) und genauer durch den Übergang des Kolbens (18) in eine vorgegebene Position ausgelöst werden.

7. Vorrichtung nach Anspruch 5 oder 6, bei der die Speichereinrichtungen (26) einen Anschlag (34, 42) zum Speichern des Implantats umfassen, der eine aktive Speicherposition, bevor die Nadel (16) in die Eindringposition gelangt, und eine inaktive Position, sobald die Nadel sich in der Eindringposition befindet, einnehmen kann.

8. Vorrichtung nach einem der vorangehenden Ansprüche, bei der der Speicheranschlag (34, 42) von einer elastischen Klammer (30, 32) gehalten wird.

9. Vorrichtung nach einem der Ansprüche 5 bis 8, bei der die Speichereinrichtungen durch ein Speicherelement (26) gehalten werden und die Deaktivierung einerseits ein Zusammenwirken zwischen dem Speicherelement (26) und dem Aufnahmegehäuse (12) für das Implantat und andererseits ein Zusammenwirken zwischen dem Speicherelement (26) und dem Kolben (18) umfasst.

10. Vorrichtung nach einem der Ansprüche 5 bis 9, bei der die Deaktivierungseinrichtungen (38, 40; 44, 46) so ausgelegt sind, dass sie ein radiales Spreizen der Speichereinrichtungen (26) bewirken.

11. Vorrichtung nach einem der Ansprüche 1 bis 10 mit Verifikationseinrichtungen (17, 104), die derart ausgelegt sind, dass das Implantat (22) für einen Benutzer sichtbar ist, wenn es von den Speichereinrichtungen (26, 102) gespeichert wird, insbesondere wenn die Nadel (16) sich in einer Position befindet, in der sie für den Benutzer nicht sichtbar ist.
